# EUROPEAN PATENT SPECIFICATION

(11) **EP 1 743 601 B1**
(45) Date of publication and mention of the grant of the patent: **05.11.2008**
(21) Application number: 06116307.7
(22) Date of filing: 29.06.2006
(51) Int. Cl.: A61F 2/16

(54) **Intraocular lens system**
Intraokularlinsensystem
Système de lentilles intraoculaires

(30) Priority: 11.07.2005 US 178758
(43) Date of publication of application: 17.01.2007
(73) Proprietor: Alcon, Inc., 6331 Hünenberg (CH)
(72) Inventor: Soye, Paul J., Fort Worth, TX Texas 76107 (US); Zhang, Xiaoxiao, Forth Worth, TX Texas 76132 (US)
(74) Representative: Hanna, Peter William Derek

(56) References cited:
- EP-A- 0 941 717
- DE-A1- 10 059 482
- US-A- 5 713 958
- US-A- 5 824 074
- US-A1- 2004 024 454
- US-A1- 2005 015 145
- US-A1- 2006 069 432
- US-B1- 6 616 691

## Description

### Background of the Invention

This invention relates generally to the field of intraocular lenses (IOL) and, more particularly, to multi-lens, micro-incision IOLs.

The human eye in its simplest terms functions to provide vision by transmitting light through a clear outer portion called the cornea, and focusing the image by way of a crystalline lens onto a retina. The quality of the focused image depends on many factors including the size and shape of the eye, and the transparency of the cornea and the lens.

When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the lens function by an artificial intraocular lens (IOL).

In the United States, the majority of cataractous lenses are removed by a surgical technique called phacoemulsification. During this procedure, an opening is made in the anterior capsule and a thin phacoemulsification cutting tip is inserted into the diseased lens and vibrated ultrasonically. The vibrating cutting tip liquifies or emulsifies the lens so that the lens may be aspirated out of the eye. The diseased lens, once removed, is replaced by an artificial lens.

Prior to the present invention, when a cataract or other disease required the removal of the natural lens and replacement with an artificial IOL, the IOL was a monofocal lens. Most IOLs are sold in power increments of +/- 0.5 diopters, and the ultimate power of the lens depends upon where the lens sits along the optical axis. The fixed increment of the lens, and the slight variation in lens placement can result in less than optimum vision. Although this situation occurs relatively infrequently, and generally is not severe, some patients ultimately are required to use a pair of spectacles or contact lenses for optimum vision. If the power of the implanted lens is incorrect, removal and exchange of a new lens is difficult because of fibrosis of the lens haptics within the capsular bag.

There have been several prior suggested adjustable power IOLs, none of which have been commercially introduced. For example, U.S. Pat. No. 5,222,981 (Werblin) and U.S. Pat. No. 5,358,520 (Patel), suggest the use of a second or even a third optic that may be implanted and attached to a previously implanted primary optic so as to adjust the overall optic power of the multi-lens system. U.S. Pat. Nos. 5,628,798 and 5,800,533 (Eggleston, et al.), and EP-A-0,941,717 (Anello, et al), disclose a threadedly adjustable IOL wherein the location of the optic along the visual axis may be adjusted. U.S. Pat. No. 4,575,373 (Johnson), discloses an IOL having an optic and an outer ring and connections between the optic and the outer ring made from a heat-shrinkable plastic. The connections are heated with a laser to adjust the power of the IOL. U.S. Patent Nos. 4,919,151 and 5,026,783 (Grubbs, et al.) disclose a lens made from a polymer that swells or otherwise changes shape. The lens is implanted or injected into the capsule bag and selectively polymerized so as to adjust the power of the optic. U.S. Patent No. 5,571,177 (Deacon, et al.), discloses an IOL having haptics with frangible stiffeners. Once implanted in an eye, the stiffeners are selectively cut or heated above their t_{g} by laser radiation, causing the stiffness of the haptic to change and adjusting the location of the lens within the capsule bag. The multi-lens designs and the threadedly adjustable designs are not optimized for the reduction or elimination of posterior capsule opacification (PCO). In addition, many of these lenses are not capable of being implanted through a vary small (less than 2 millimeters) incision.

Document US 2005/0015145 discloses the features of the preamble of claim 1. Therefore, a need continues to exist for a safe and stable intraocular lens system that provides adjustment of lens power. Such a lens system could be used in cataract or clear lens exchange surgeries.

### Brief Summary of the Invention

The present invention improves upon the prior art by providing a two or three component lens system in accordance with claims which follow. The first component is a ring-like supporting component that is implanted in the capsular bag following cataract surgery. The first component is a non-optical component and does not correct for any refractive errors. The first component may contain features to help reduce or eliminate PCO. The second component is an optical component that may contain all of the corrective optical power of the lens system. The second component has a pair of tabs for locking the second component within the first component. The first component includes a feature that allows the surgeon to change the position of the second component along the optical axis of the lens system. The third component is optional and is similar to second component and contains some optical power to correct for any residual optical error not corrected by the second component. The second and third components may also be implanted so as to move relative to one another, thereby providing some accommodation.

Accordingly, one objective of the present invention is to provide a safe and biocompatible intraocular lens.

Another objective of the present invention is to provide a safe and biocompatible intraocular lens that is easily implanted in the posterior chamber.

Still another objective of the present invention is to provide a safe and biocompatible intraocular lens that is stable in the posterior chamber.

Still another objective of the present invention is to provide a safe and biocompatible adjustable lens system.

Still another objective of the present invention is to provide a safe and biocompatible lens system that can be implanted through a small incision.

Still another objective of the present invention is to provide a safe and biocompatible lens system that helps reduce the incidence of PCO.

Still another objective of the present invention is to provide a safe and biocompatible lens system for use in cataract and/or clear lens exchange surgeries.

These and other advantages and objectives of the present invention will become apparent from the detailed description and claims that follow.

### Brief Description of the Drawing

FIG. 1 is an enlarged perspective view of a first component of the lens system of a system, which ist not part of the invention.
FIG. 2 is an enlarged plan view of the first component of the lens system, which is not part of the present invention.
FIG. 3 is an enlarged cross-sectional view of the first component of the lens system taken at line 3-3 in FIG. 2, which is not part of the invention.
FIG. 4 is an enlarged perspective view of the second component of the lens system of the present system.
FIG. 5 is an enlarged plan view of the second component of the lens system of the present system.
FIG. 6 is an enlarged cross-sectional view of the second component of the lens system of the present system taken at line 6-6 in FIG. 5.
FIG. 7 is an enlarged plan view of a third component of the lens system, which is not part of the present invention.
FIG. 8 is an enlarged cross-sectional view of the third component of the lens system of the present system taken at line 8-8 in FIG. 7.
FIG. 9 is an enlarged cross-sectional view of the lens system with the second component installed within the first component, which is not part of the present invention.
FIG. 10 is an enlarged plan view of the first component of the lens system in accordance with the present invention.

### Detailed Description of the Invention

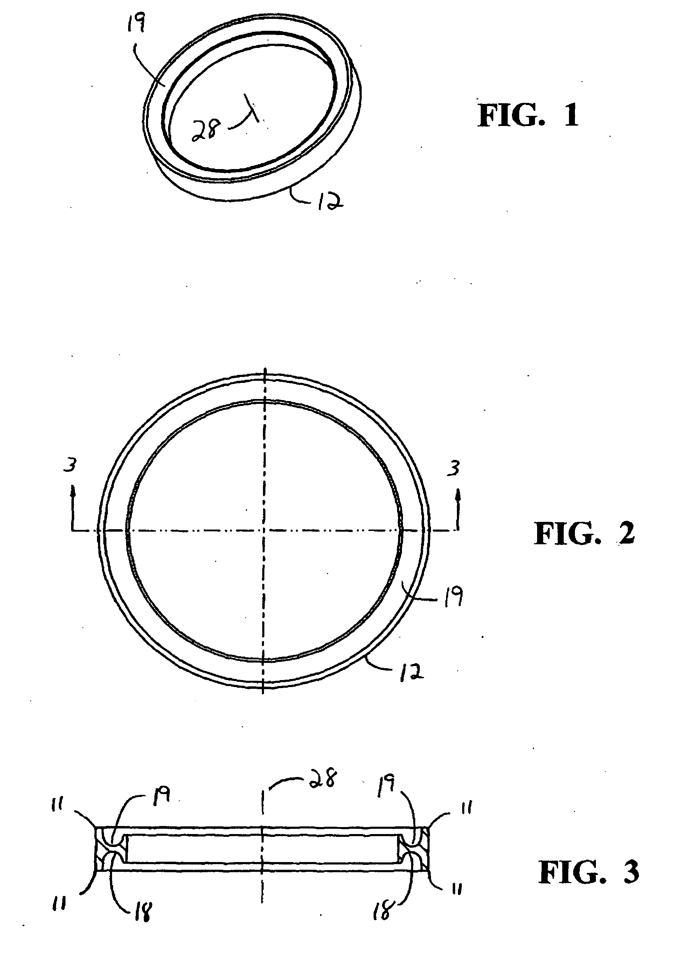

As best seen in FIGS. 1, 4 and 7, lens system 10 may generally' include a first, or base, component 12, second, or optical, component 14 and may optionally include third, or secondary optical component 16. First component 12 is generally ring-like, and, as best seen in FIG. 3, is generally "I"-shaped in cross section. This "I"-shape forms circumferential anterior channel 19 and posterior channel 18 within the inner diameter of component 12. Such a construction is easy to mold, and provides the flexibility necessary to allow component 12 to be inserted into an eye through a sub-2 millimeter incision. Component 12 is constructed with sharp, square outer edges 11 to help prevent PCO. Component 12 is preferably formed in any suitable overall diameter, for example, between approximately 8.0 millimeters and 12.0 millimeters, a suitable interior diameter, for example, between approximately 6.0 millimeters and 8.5 millimeters and made from a soft, foldable material such as a soft acrylic. Alternatively, component 12 may be made from a material that is stiffer relative to optical component 14 or less stiff relative to optical component 14. By way of example, component 12 may be made of rubber elastomers, such as butyl rubber, latex rubber, natural rubber, pure gum rubber, neoprene rubber, acrylonitrile rubber, styrene-butadiene rubber, ethylene-propylene diene monomer rubber, acrylonitrile-butadiene-styrene (ABS) rubber, epichlorohydrin rubber, hypalon rubber, silicone rubber and siloxane elastomers, such as poly(dimethylsiloxane), polyurethane rubber, viton rubber, ethylene-butylene rubber, isobutylene rubber and elastomers of polyphosphazenes, like poly(bis-trifluorethoxyphosphazene) oly(dimethylphosphazene) and poly(phenylmethylphosphazene). Preferably, base component 12 may be formed so as to be opaque, such as by frosting or texturing the anterior and/or posterior surfaces of base component 12, or base component may be relatively clear. Base component 12 may also contain a chromophore to block ultraviolet and/or blue and/or green light, such chromophore(s) being well-known in the art.

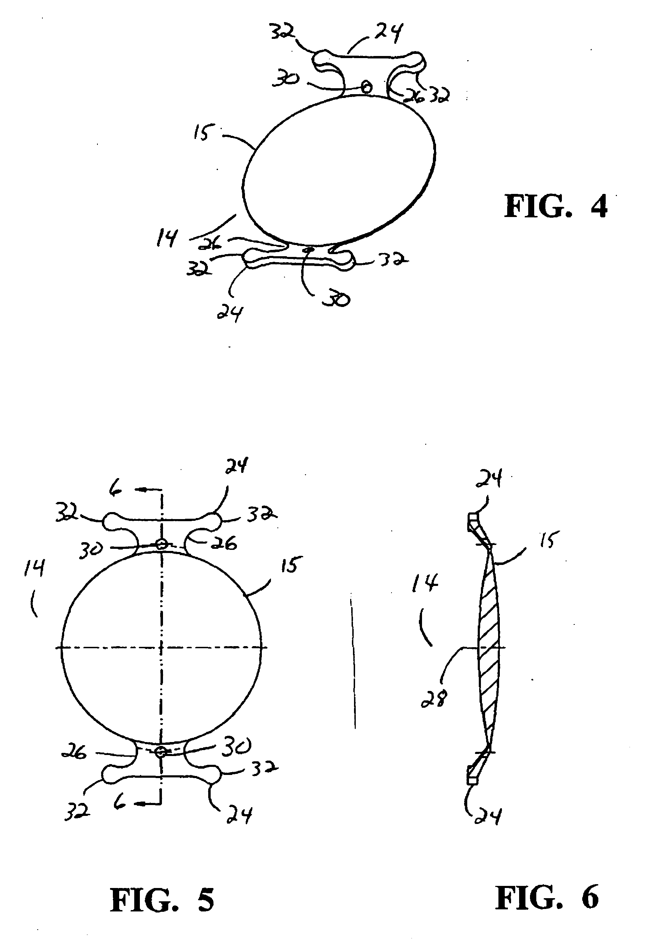

As best seen in FIGS. 4-6, second component 14 is generally circular with an optic 15 having a diameter for example, between approximately 4.0 millimeters and 7.0 millimeters. Optic 15 tapers from being relatively thick in the middle to having a relatively thin, or sharp, edge that connects to a plurality of haptics 24 integrally formed with optic 15 so as to give optical component 14 overall length of between approximately 8.0 millimeters and 10.0 millimeters and preferably, is made from a soft, foldable material such as a soft acrylic. Second component 14 may also contain a chromophore to block ultraviolet and/or blue light, such chromophore(s) being well-known in the art, but unlike base component 12, second component 14 is optically clear. Haptics 24 are connected to optic 15 by connecting portions 26 that are relatively wide in plan view, but relatively thin in cross-section. In addition, haptics 24 contain outwardly projecting tips 32. Such a construction helps to prevent rotation of second component 14 within first component 12 and helps to maintain the stability of optical portion 14 in the plane perpendicular to optical axis 28, but allows some flexibility along optical axis 28. Connecting portions 26 may also contain positioning or manipulation holes 30.

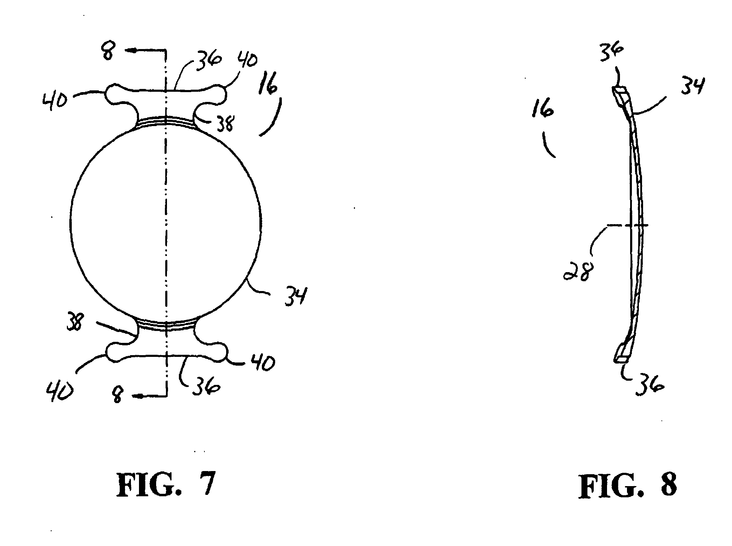

As best seen in FIGS. 7-8, third component 16 is generally circular with an optic 34 having a diameter for example, between approximately 4.0 millimeters and 7.0 millimeters. Third component 16 contains a plurality of haptics 36 integrally formed with optic 34 so as to give third component 16 overall length of between approximately 8.0 millimeters and 10.0 millimeters and preferably, is made from a soft, foldable material such as a soft acrylic. Third component 16 may also contain a chromophore to block ultraviolet and/or blue light, such chromophore(s) being well-known in the art, but unlike base component 12, lens component 16 is optically clear. Haptics 36 are connected to optic 34 by connecting portions 38 that are relatively wide in plan view, but relatively thin in cross-section. In addition, haptics 36 contain outwardly projecting tips 40. Such a construction helps to prevent rotation of third component 16 within second component 12 and helps to maintain the stability of third component 16 in the plane perpendicular to optical axis 28, but allows some flexibility along optical axis 28. In general, third component 16 is of similar construction as second component 14 except, as best seen in FIGS. 6 and 8, third component 16 has less optical power than second component 14 and therefore, is generally thinner than second component 14. Either second component 14 or third component 16 may be constructed to correct any of a variety of possible refractive errors, such a astigmatism (toric), presbyopia (accommodative, pseudo-accommodative or multifocal) or customized to correct higher order aberrations, such refractive errors and optical corrections therefore being well-known in the art.

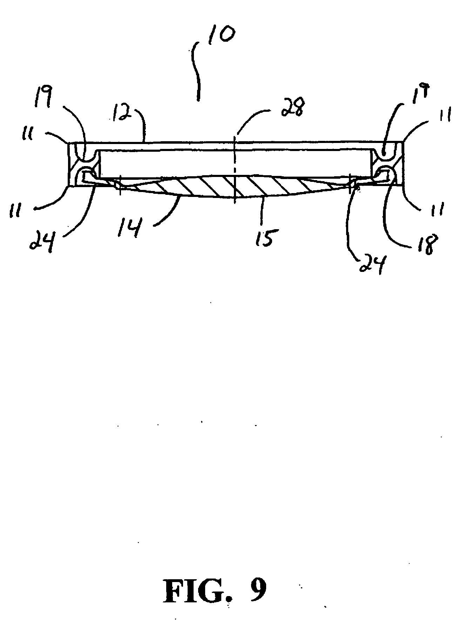

As best seen in FIG. 9, lens system 10 is assembled by placing tips 32 or 40 of second component 14 or third component 16, respectively, into posterior channel 18 of first component 12, thereby compressing connecting portions 26 and 38 respectively and allowing both haptic 24 and 36 to snap within channel 18. Third component 16 may be installed in a similar manner to correct any residual refractive errors not corrected by second component 14. Preferably, third component 16 is rotated approximately 90° relative to second component 14.

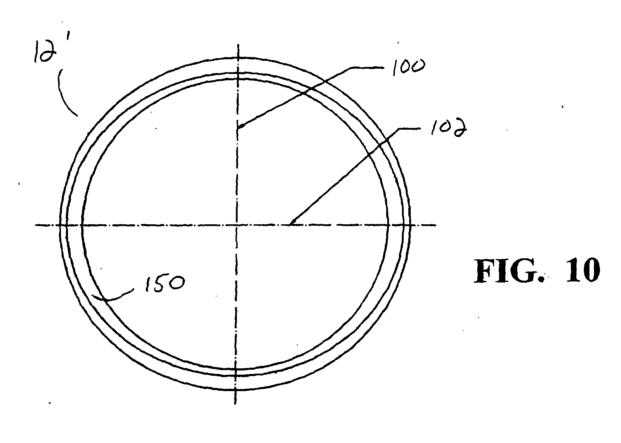

As best seen in FIG. 10, in an embodiment of the present invention, first or base component 12' has an inner rim or lip 150 that is slightly elliptical, oval, out-of-round or non-circular in shape having a short axis 100 and a long axis 102. This slightly oval or otherwise out of round shape will vary the amount of compression on haptics 24 of second component 14. Rotation of second component 14 so that haptics 24 are aligned along short axis 100 will cause haptics 24 to more compressed than rotation of second component 14 so that haptics 24 are aligned along long axis 102, thereby changing the position of optic 15 along optical axis 28 and correspondingly varying the effective refractive power of lens system 10 implanted in an eye.

This description is given for purposes of illustration and explanation. It will be apparent to those skilled in the relevant art that changes and modifications may be made to the invention described above without departing from its scope.

## Claims

1. An intraocular lens system (10), comprising:
a) a ring-like first component (12') having an inner rim or lip (150, 200); and
b) a second component (14) having an optic portion (15) with an optical power, the second component having a plurality of haptics (24) sized to ride on the inner rim or lip of the first component, so that rotation of the second component causes the second component to move along the optical axis (28) of the lens system and vary the effective power of the lens system,
**characterized in that**
the inner rim or lip (150) has a slightly elliptical, oval, out-of-round or non-circular shape in plan view with a short axis (100) and a long axis (102), adapted to vary the amount of compression on the haptics (24) of the second component, (14) wherein rotation of the second component (14) so that the haptics are aligned along the short axis will cause the haptics to be more compressed than rotation of the second component so that the haptics are aligned along the long axis, thereby causing the position of the optic portion (15) of the second component to change along the optical axis (28), while maintaining the stability of the optic portion of the second component in a plane perpendicular to the optical axis.

2. The lens system of claim 1, wherein the first component (12') is opaque.

3. The lens system of claim 1, wherein the first component (12') is stiff relative to the second component (14).

4. The lens system of claim 1, wherein the first component (12') is made from a rubber elastomer.

5. The lens system of claim 4, wherein the rubber elastomer is selected from; butyl rubber, latex rubber, natural rubber, pure gum rubber, neoprene rubber, acrylonitrile rubber, styrene-butadiene rubber, ethylene-propylene diene monomer rubber, acrylonitrile-butadiene-styrene (ABS) rubber, epichlorohydrin rubber, hypalon rubber, silicone rubber and siloxane elastomers including poly(dimethylsiloxane), polyurethane rubber, viton rubber, ethylene-butylene rubber, isobutylene rubber and elastomers of polyphosphazenes including poly(bis-trifluorethoxyphosphazene), poly(dimethylphosphazene) or poly(phenylmethylphosphazene).

6. The lens system of claim 1, wherein the second component (14) contains a chromophore to block ultraviolet and/or blue and/or green light.

7. The lens system of claim 1, wherein the first component (12') is made of a soft acrylic.

8. The lens system of any one of claims 1 to 7, wherein the second component (14) is constructed to correct astigmatism.

9. The lens system of any one of claims 1 to 7, wherein the second component (14) is customized to correct higher order aberrations.

## Patentansprüche

1. Intraokularlinsensystem (10), welches enthält:
a) ein ringförmiges erstes Bauteil (12'), welches eine Innenkante oder einen Rand (150, 200) hat; und
b) ein zweites Bauteil (14), welches einen Optikabschnitt (15) mit einer optischen Leistung hat, wobei das zweite Bauteil eine Mehrzahl von Haptiken (24) hat, welche derart bemessen sind, dass sie auf der Innenkante oder dem Rand von dem ersten Bauteil verlaufen, so dass eine Umdrehung von dem zweitem Bauteil bewirkt, dass sich das zweite Bauteil entlang der optischen Achse (28) von dem Linsensystem bewegt und die Wirkleistung von dem Linsensystem variiert,
**dadurch gekennzeichnet, dass**
die Innenkante oder der Rand (150) eine leicht elliptische, ovale, vom Rundlauf abweichende oder unrunde Form in der Draufsicht mit einer kurzen Achse (100) und einer langen Achse (102) hat, welche dazu ausgelegt ist, um die Kompressionsgröße von den Haptiken (24) von dem zweiten Bauteil (14) zu variieren, wobei eine Umdrehung des zweiten Bauteils (14), so dass die Haptiken entlang der kurzen Achse ausgerichtet sind, bewirken wird, dass die Haptiken stärker komprimiert sind als bei einer Umdrehung des zweiten Bauteils derart, dass die Haptiken entlang der langen Achse ausgerichtet sind, wodurch bewirkt wird, dass sich die Position von dem Optikabschnitt (15) von dem zweiten Bauteil entlang der optischen Achse (28) ändert, während die Stabilität von dem Optikabschnitt von dem zweiten Bauteil in einer Ebene senkrecht zur optischen Achse beibehalten ist.

2. Linsensystem nach Anspruch 1, bei welchem das erste Bauteil (12') lichtundurchlässig ist.

3. Linsensystem nach Anspruch 1, bei welchem das erste Bauteil (12') in Relation zum zweiten Bauteil (14) versteift ist.

4. Linsensystem nach Anspruch 1, bei welchem das erste Bauteil (12') aus einem Kautschuk-Elastomer erstellt ist.

5. Linsensystem nach Anspruch 4, bei welchem das Kautschuk-Elastomer ausgewählt ist aus: Butylkautschuk, Latexkautschuk, natürlicher Kautschuk, reiner Gummikautschuk, Neoprenkautschuk, Acrylnitrilkautschuk, Styrolbutadienkautschuk, Ethylen-Propylen-Dien-Monomerkautschuk, AcrylnitrilButadien-Styrol(ABS)-Kautschuk, Epichlorhydrin-Kautschuk, Hypalon-Kautschuk, Silikonkautschuk und Siloxan-Elastomere, welche Poly(dimethylsiloxan), Polyurethan-Kautschuk, Viton-Kautschuk, Ethylen-Butylen-Kautschuk, Isobutylen-Kautschuk und Elastomere aus Polyphosphazenen enthalten, welche Poly(bis-trifluorethoxyphosphazen), Poly(dimethylphosphazen) oder Poly(phenylmethylphosphazen) enthalten.

6. Linsensystem nach Anspruch 1, bei welchem das zweite Bauteil (14) einen Chromophor enthält, um ultraviolettes und/oder blaues und/oder grünes Licht zu blockieren.

7. Linsensystem nach Anspruch 1, bei welchem das erste Bauteil (12') aus einem nachgiebigen Acryl erstellt ist.

8. Linsensystem nach einem der Ansprüche 1 bis 7, bei welchem das zweite Bauteil (14) derart aufgebaut ist, um Astigmatismus zu korrigieren.

9. Linsensystem nach einem der Ansprüche 1 bis 7, bei welchem das zweite Bauteil (14) dazu gefertigt ist, um Abbildungsfehler einer höheren Ordnung zu korrigieren.

## Revendications

1. Système de lentille intraoculaire (10), comportant :
a) un premier composant analogue à un anneau (12') ayant un rebord intérieur ou une lèvre intérieure (150, 200), et
b) un second composant (14) ayant une partie optique (15) dotée d'une puissance optique, le second composant ayant une pluralité d'haptiques (24) dimensionnées pour chevaucher le rebord intérieur ou la lèvre intérieure du premier composant, de sorte qu'une rotation du second composant amène le second composant à se déplacer le long de l'axe optique (28) du système de lentille, et modifie la puissance effective du système de lentille,
**caractérisé en ce que**
le rebord intérieur ou la lèvre intérieure (150) a une forme légèrement elliptique, ovale, non ronde ou non circulaire en vue de dessus, avec un axe court (5) et un axe long (102), adaptée pour modifier la quantité de compression sur les haptiques (24) du second composant (14), une rotation du second composant (14) de manière à ce que les haptiques soient alignées le long de l'axe court amenant les haptiques à être comprimées davantage qu'une rotation du second composant, de manière à ce que les haptiques soient alignées le long de l'axe long, en amenant ainsi la position de la partie optique (15) du second composant à changer le long de l'axe optique (28), tout en maintenant la stabilité de la partie optique du second composant dans un plan perpendiculaire à l'axe optique.

2. Système de lentille selon la revendication 1, dans lequel le premier composant (12') est opaque.

3. Système de lentille selon la revendication 1, dans lequel le premier composant (12') est rigide par rapport au second composant (14).

4. Système de lentille selon la revendication 1, dans lequel le premier composant (12') est réalisé en élastomère de caoutchouc.

5. Système de lentille selon la revendication 4, dans lequel l'élastomère de caoutchouc est sélectionné parmi : du caoutchouc de butyle, du caoutchouc de latex, du caoutchouc naturel, du caoutchouc de gomme pure, du caoutchouc de néoprène, du caoutchouc d'acrylonitrile, du caoutchouc de styrène-butadiène, du caoutchouc de monomère d'éthylène-propylène diène, du caoutchouc d'acrylonitrile-butadiène-styrène (ABS), du caoutchouc d'épichlorhydrine, du caoutchouc d'Hypalon, du caoutchouc de silicone et des élastomères de siloxane comprenant du poly(diméthylsiloxane), du caoutchouc de polyuréthanne, du caoutchouc de Viton, du caoutchouc d'éthylène-butylène, du caoutchouc d'isobutylène et des élastomères de polyphosphazènes comprenant du poly(bis-trifluoroéthoxyphosphazène), du poly(diméthylphosphazène) ou du poly(phénylméthylphosphazène).

6. Système de lentille selon la revendication 1, dans lequel le second composant (14) contient un chromophore pour bloquer une lumière ultraviolette et/ou bleue et/ou verte.

7. Système de lentille selon la revendication 1, dans lequel le premier composant (12') est constitué d'un acrylique mou.

8. Système de lentille selon l'une quelconque des revendications 1 à 7, dans lequel le second composant (14) est réalisé pour corriger un astigmatisme.

9. Système de lentille selon l'une quelconque des revendications 1 à 7, dans lequel le second composant (14) est réalisé sur mesure pour corriger des aberrations d'ordre supérieur.
